# EUROPEAN PATENT APPLICATION

(11) **EP 3 656 418 A1**
(43) Date of publication of application: **27.05.2020**
(21) Application number: 18207790.9
(22) Date of filing: 22.11.2018
(51) Int. Cl.: A61M 5/145, A61M 5/315

(54) **AN INJECTION DEVICE COMPRISING LENGTH AND VOLUME ADAPTERS**

(71) Applicant: Tecpharma Licensing AG, 3401 Burgdorf (CH)
(72) Inventor: Burren, Stefan, 3150 Schwarzenburg (CH); Bernhard, Mario, 3400 Burgdorf (CH); Mellenberger, Andres, 3425 Koppigen (CH); Tschirren, Markus, 3400 Burgdorf (CH); Schenker, Susanne, 4912 Aarwangen (CH); Schrul, Christian, 3400 Burgdorf (CH)

(57) **Abstract**

An injection device comprising a housing comprising a cartridge received in a cartridge holder and the cartridge contains a medicament sealed in a barrel defining a longitudinal axis and the ends of the barrel are closed by a moveable plunger and a pierceable septum. The injection device further comprises a drive assembly having a curved piston rod configured for advancement of the plunger in the cartridge along the longitudinal axis from a retracted position to an extended position for expelling the medicament from the injection device. An interchangeable adapter is positioned between the curved piston rod and the plunger for transferring the load from the piston rod to the plunger during advancement, and a pivot bearing is formed between the end of the piston rod and the adapter

## Description

### TECHNICAL FIELD

The current invention relates to an injection device comprising a cartridge and adapters to accommodate different cartridges into the injection device. Furthermore a method is presented for assembling the injection device.

### BACKGROUND OF THE INVENTION

Injection devices are used for the subcutaneous or intermuscular delivery of liquid medicaments to a patient. Infusion devices deliver the medication from a cartridge using a drive mechanism and a control mechanism that controls the advancement of a plunger present in the cartridge containing the medication. The medication is delivered to the patient via a fluid path and an external infusion set comprising a needle for that can be inserted into the skin. With such infusion devices both continuous and temporary profiles can be programmed for medicament delivery.

A patch device is an example of an injection or infusion device that is attachable to the skin of the patient. Such patch devices do not need an external infusion set for delivery, as the needle is directly contained in the patch device and inserted into the patient therefrom. A patch injection device delivers the medicament to the patient in a shorter time, typically less than an hour whereas a patch infusion device intends to deliver the medicament over a longer time, typically more than one day.

The patch injection devices comprise a dose setting mechanism, a delivery mechanism which is connected or connectable to a drive mechanism; a needle insertion and retraction mechanism, or a needle shield protection system. The delivery mechanism operates a piston rod which is advanced in the cartridge and thereby advances the plunger for expelling medication from the device

For the piston rod there are several options such as a linear piston rod, a (permanently) curved piston rod and a flexible piston rod that can go back and forth from a linear configuration to a curved configuration and still have the ability to transfer load for advancing the plunger in the cartridge. The advantage of the curved configuration is that space saving designs can be used thereby reducing the outer dimensions of the device. An example of a patch injection device having a piston rod that can go from a linear to a curved (U-shaped) configuration is presented in WO2017219156.

Such patch devices can be used for the delivery of a wide range of medications (see definition section below) that are available in different containers, for example in cartridges, each having different dimensions in terms of length, diameter and shoulder position. Also the fill volume for the medicament in the cartridge can be varied, thus leading to different plunger positions in the barrel forming the cartridge. The drive mechanism with the piston rod may define a fixed position for the piston rod and therefore solutions have been presented using adapters that compensate for the gap present between the piston rod and the plunger.

Such a length adapter is presented in WO2016075254 for manufacturing a range of autoinjector pens using a unified drive mechanism with a defined position of the piston rod in combination with cartridges having different fill volumes (and plunger positions). The piston rod used in the autoinjector pens is a linear piston rod that cannot be bent. Another example of a length adapter is presented in WO2006106295 for a spring driven autoinjector pen having a linear piston rod.

The cartridge holder for such devices preferably has defined axial and lateral dimensions and smaller cartridges can be inserted in a unified cartridge holder using an adapter to compensate for a lateral clearance. In US9427519, a monolithic adapter is shown configured to receive cartridges of different sizes for pumping fluid into a body. The adapter is presented together with a telescopic drive mechanism that is axially aligned with cartridge axis.

Piston rods that can be bent to go from a linear shape to a U-shape and back to a linear shape comprise segments that are connected to each other via hinges. When the piston rod transforms back from the U-shape to a linear shape, then segments that are adjacent to each other need to move back to a straight configuration and the hinges need to rotate. A disadvantage is that not all hinges rotate sufficiently and the piston rod may enter the cartridge tilted with respect to the longitudinal axis of the cartridge such that the plunger is not loaded in the center of the cartridge potentially leading to tilting or leakage of the plunger.

It is an object of the present invention to provide an injection device having a unified drive mechanism and a unified cartridge holder that is configured to receive a variety of cartridges in terms of fill volumes and outer dimensions. The unified drive mechanism uses a piston rod that can be bent from a linear to a curved configuration and back to a linear configuration. A versatile injection device is provided that serves as a platform for a wide variety of cartridges that can be operated safe and robust in combination with a segmented and bendable piston rod. Furthermore it is an objective to present an assembly method for the injection device.

These objectives are solved by providing an injection device having an interchangeable adapter that compensates for the gap between the piston rod and the plunger in the cartridge and the adapter forms a pivot bearing between the end of the piston rod and the adapter for a homogeneous loading of the plunger. Furthermore, a volume adapter is provided in the dependent claims to increase the versatility of the injection device. A method for assembling the injection device with the interchangeable adapter to form the pivot bearing is presented as well.

### DEFINITIONS

The term "medicament" or "medication" includes any flowable medical formulation suitable for controlled administration through a means such as, for example, a cannula or a hollow needle and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from -or harvested by- biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The distal end or distal direction is defined by the direction of the needle configured to penetrate the skin of the patient. For an injection pen this may be the injection needle and the end of the pen holding the needle or being configured to hold the needle is the distal end. For an infusion device the distal end and the distal direction is towards the needle configured to penetrate the skin of the patient, which may be along the axis of the device or tilted or perpendicular to the axis of the device. The distal direction in an infusion device represents the direction in which the medicament flows towards the insertion needle. The proximal direction or end is opposite to the distal direction or end.

The mere fact that certain elements or steps are recited in distinct claims shall not preclude the existence of further meaningful combinations of these elements or steps.

### GENERAL DESCRIPTION OF THE INVENTION

The invention relates to an injection device that comprises a housing comprising a cartridge holder. The injection device has a cartridge received in the cartridge holder which contains a medicament sealed in a barrel defining a longitudinal axis. The ends of the barrel are closed by a moveable plunger and a pierceable septum, and the medicament is sealed there between. A drive assembly is part of the injection device which comprises a curved piston rod, configured for advancement of the plunger in the cartridge along the longitudinal axis. The piston rod moves from a retracted position to an extended position for expelling the medicament from the cartridge in the injection device. The injection device further comprises an interchangeable adapter that is positioned between the end of the piston rod and the plunger in the cartridge for transferring the load from the piston rod to the plunger during advancement, wherein a pivot bearing is formed between the end of the piston rod and the interchangeable adapter. The curved piston rod should be understood as a piston rod that is temporarily curved and preferably changes from a linear configuration into a curved, or bent, configuration before going back into the linear configuration and entering the cartridge. The drive assembly is preferably adapted to advance the piston rod or segments of the piston rod. The drive mechanism preferably advances a part of the piston rod that is in the linear configuration and during advancement the piston rod is forced to change into the curved configuration, for example by guiding elements present in the housing, for example using a curved guiding section in the housing. Optionally, the piston rod is forced to go back into the linear configuration, for example by a linear guiding element provided by the housing. Curved piston rods may comprise segments that are connected to each other and during the advancement of the piston rod the segments are bent for making a U-turn (curved configuration) and after the U-turn, the segments need to be aligned in a stacked position for entering the barrel of the cartridge requiring relative rotation subsequent segments. Often the segments are not completely returned to the stacked position and thereby increasing the risk of non-uniform, or off axis loading of the plunger having the risk of tilting the plunger or increasing the risk of leakage of medicament. The interchangeable adapter forms a pivot bearing between the back end of the adapter and the distal end of the piston rod in order to equally distribute the force from the piston rod to the plunger and compensate for off-axis, or non-uniform loading and therefore reduce the tilting/leakage of the plunger.

Preferably, the end of the piston rod has a fixed position with respect to the housing when the piston rod is in the retracted position and the cartridge holder is available for receiving a cartridge. The fixed position implies that one unified housing having a unified drive assembly with a piston rod at a fixed position can be combined with different cartridges by using the interchangeable adapter and have enough space available for inserting the cartridge into the holder.

The injection device may further comprise a volume adapter additional to the interchangeable adapter. The volume adapter is located in the cartridge holder of the injection or infusion device. The cartridge holder defines a first space having a first volume, preferably inner volume. The volume adapter comprises a body, preferably cylindrically shaped, defining a second space that is to be arranged and fits within the first volume of the cartridge holder. Preferably the second space that is occupied by the external dimensions of the body fits within the first volume of the cartridge holder. There may be a small clearance between the body, or outside surface of the body and the cartridge holder to facilitate the insertion of the body into the cartridge holder. Preferably, the clearance in length and diameter for a cylindrical shaped body in the cartridge holder is below 5% of the length or diameter of the body, more preferably below 1% of those dimensions. The body of the volume adapter comprises a hollow interior, preferably cylindrically shaped defining a third space having a third volume that is below the first volume wherein the cartridge may be received within the hollow interior. Thus the outer dimensions of the cartridge fit into the inner dimensions of the hollow interior having the advantage that one cartridge holder (having fixed inner dimensions) can accommodate cartridges with a third volume that is below the first volume such that the volume adapter can be used with a plurality of cartridges having different outer dimensions without the need for a separate cartridge holder that can accommodate each specific cartridge. A range of volume adapters having fixed outer dimensions and a range of inner dimensions for the hollow interior may be provided to improve the versatility of the injection device.

The volume adapter or the body forming the volume adapter comprises two open ends, a first open end for receiving the cartridge and a second open end, located opposite to the first open end, defining a passage for a needle.

The injection device has a segmented piston rod having connectors, preferably an integral hinge or a living hinge, between the individual segments allowing for relative rotational movement between the individual segments. The hinges allow the piston rod to make a curve and thereby save space and can bend or make the U-turn. The connectors allow for relative rotational back and forth movement between the segments such that the segmented piston rod can go from a straight to a curved configuration and vice versa from a curved to a straight configuration.

The interchangeable adapter has a length calculated from the length of the cartridge holder deducted by the distance between the proximal end of the plunger and the distal end of the septum. The axial dimension of the cartridge holder defines a cartridge holder length and by deducting this length by the distance between the plunger and septum in the cartridge, the length for the interchangeable adapter is obtained. The length of the adapter is varied and adapted to the fill volume of the cartridge such that one unified drive mechanism can be combined with different cartridges having different positions of the plunger without having to advance the piston rod (e.g. factory priming) for establishing the contact between the piston rod and the plunger as the interchangeable adapter compensates for the gap. The sum of the length of the interchangeable adapter and the distance between the proximal end of the plunger and the distal end of the septum is always identical for each combination of a cartridge and interchangeable adapter and preferably equals the axial length of the cartridge holder. Optionally, the length of the interchangeable adapter is calculated by deducting an extra distance (related to a fill finish gap) from the length of the cartridge holder. The extra distance may be required to have a safe assembly of the plunger and adapter as it is not desired that the proximal surface of the plunger is face with the proximal end of the cartridge. The fill-finish gap ensures that the plunger is always positioned inside the cartridge.

The interchangeable adapter preferably has a cylindrical shape with an outer diameter that is below the inner diameter of the barrel of the cartridge, preferably having an outer diameter that is at least 85 % of the inner diameter of the barrel, more preferably at least 95% of the inner diameter. The relatively tight fit between the interchangeable adapter and the wall of the cartridge prevents tilting of the adapter and correct load transfer from the piston rod to the plunger.

The interchangeable adapter has a front end configured for engaging with, or connecting to the plunger in the cartridge. For example the distal end of the adapter may have a protrusion corresponding a matching recessed section or bore in the plunger, alternatively a screw type of connection may be envisaged. An efficient load transfer from the interchangeable adapter to the plunger prevents tilting between the front end of the adapter and back end of the plunger.

The pivot bearing is formed between the distal end of the piston rod and a back end of interchangeable adapter, thus compensating for off axis loading or non-uniform loading of the plunger. The pivot bearing may comprise a protrusion present at the end of the piston rod or on the back end of the interchangeable adapter, preferably the protrusion has a convex shape. The protrusion interacts with at least one flat surface present on the piston rod or the adapter when the interchangeable adapter is inserted into the cartridge which itself has been inserted into the cartridge holder. The pivot bearing is preferably a ball and plate bearing established upon cartridge insertion. Alternatively, the pivot bearing comprises one convex shaped protrusion present on the piston rod or the adapter that is matched with a complementary concave shaped recession present on the piston rod or the adapter.

The last segment of the segmented piston rod may comprise a guiding means for guiding the piston rod into the barrel of the cartridge. The guiding means is for example a guiding fin oriented parallel to the longitudinal axis of the cartridge barrel which may abut the inner wall of the barrel and orienting the last segment thereby further reducing the off axis loading risk for the plunger.

An interchangeable adapter for an injection or infusion device adapted for an assembly with a piston rod and a cartridge comprising a plunger, the adapter can be positioned in the assembly between the distal end of the piston rod and the plunger. The interchangeable adapter comprises a cylindrical body defining a longitudinal axis, the cylindrical body having a length that is adjusted to the gap between the end of the piston rod and the plunger in the cartridge. The cylindrical body has a front end adapted for abutting or engaging the plunger in the cartridge and a back end that is opposite to the front end comprising means to form a pivot bearing with the end of the piston rod. The cylindrical body has an outer diameter that is below the inner diameter of the cartridge.

A volume adapter for an injection or an infusion device configured to be inserted in a cartridge holder of the injection or infusion device wherein the cartridge holder defines a first space having a first inner volume, the volume adapter comprising:
- A monolithic body, preferably cylindrically shaped defining a second space to be arranged within the first volume of the cartridge holder
- An hollow interior, preferably cylindrically shaped defining a third space having a third volume that is below the first inner volume wherein a cartridge can be received within the hollow interior,
wherein the cartridge can be removable or non-removable received by the volume adapter.

The volume adapter may be made from a transparent material such that the cartridge, and the medicament can be viewed through the volume adapter. The volume adapter may have markings printed onto the outside surface such that the user can monitor the amount of medicament that is injected. Optionally, the volume adapter may be colored using a dye or pigment to prevent light, for example UV light from entering the cartridge. In an example, the adapter is non-transparent, for example colored black.

The invention further relates to a method for assembling a range of injection device or infusion devices having cartridges with different preset medicament fill volumes and thus different positions of the plunger in the cartridges. The method comprises the following steps:
- Providing a housing comprising a cartridge holder that is located behind a window (or an opening) in the housing, the housing further encloses a drive assembly for advancing an at least partially curved piston rod that can move from a retracted position to an extended position, wherein the retracted position is defined by the axial position of the distal end of the piston rod with respect to the housing,
- Providing a cartridge containing a preset volume of a medicament, preferably selecting the cartridge from a range of cartridges with different preset medicament fill volumes. The medicament is sealed in a barrel defining a first longitudinal axis and the ends of the barrel are closed by a pierceable septum and a plunger that can be moved along the first longitudinal axis towards the pierecable septum by advancing the piston rod,
- Providing an interchangeable adapter having a defined length and which is selected from a range of interchangeable adapters having different lengths, the interchangeable adapter is selected according to the preset medicament volume of the cartridge. The length of the interchangeable adapter is calculated by deducting the distance between the plunger in the cartridge and the septum from the length of the cartridge holder (and optionally deducted by an additional fill/finish distance),

- Inserting the interchangeable adapter into the cartridge whereby a front end of the adapter engages the plunger in the cartridge,
- Inserting the cartridge comprising the interchangeable adapter through the window into the cartridge holder to form a pivot bearing between the back end of the adapter and the end of the piston rod,
- Closing the window in the housing with a cover.

Additionally, the assembly method may comprise a further step in that prior to inserting the cartridge through the window into the cartridge holder the cartridge itself is inserted into a volume adapter. The volume adapter is configured to be inserted in the cartridge holder defining a first space having a first volume, the volume adapter comprises:A monolithic body, preferably cylindrically shaped defining a second space that fits within the first space having the first volume of the cartridge holder. The body comprises a hollow interior, preferably cylindrically shaped defining a third space having a third volume that is below the first volume wherein the cartridge containing the preset volume is received within the hollow interior, or alternatively the cartridge is coaxially guided by the volume adapter when received in the hollow interior.

The cartridge can be removable or non-removable received by the volume adapter, such that either the volume adapter may be reused (removable) or it may increase the handling and prevent breakage of glass cartridges during assembly (non-removable). In the latter example, flexible hooks or a snapper or snap ring may be present at the proximal rim of the volume adapter that engage with the proximal rim of the barrel such that the cartridge cannot be removed from the volume adapter.

The cartridge holder has a longitudinal shape defining a second longitudinal axis and wherein the cartridge comprising the interchangeable adapter or the volume adapter comprising the cartridge is oriented with its first longitudinal axis parallel to the second longitudinal axis and is inserted through the window perpendicular to the first and second longitudinal axis. By orienting the cartridge parallel to the cartridge holder and subsequent insertion (parallel translation) the cartridge (with the interchangeable and/or volume adapter) is directly positioned between the distal end of the piston rod and the end of the cartridge holder. The piston rod does not have to be moved for insertion of the cartridge to make space available for the insertion step. The piston rod is in a fixed position thereby controlling the formation of the pivot bearing and making the sideways insertion of the cartridge easy.

The window in the housing is closed by a cover and preferably the cover is moved parallel to the second longitudinal axis. The housing and the cover may have engagement means for guiding the cover parallel to the second longitudinal axis during closing of the window in the housing. The window in the housing is preferably closed by irreversible connectors for example snap-fit connectors present on the cover and the housing. The cover may comprise a protrusion, wedge or crush rib that is deformed by the barrel of the cartridge or volume adapter when the window is closed by the cover thereby fixating the cartridge (or the volume adapter) in the cartridge holder along the second longitudinal axis and/or around its rotational axis. Guiding of the cover effectively controls and deforms its deformable wedge or protrusion to fixate the cartridge or the volume adapter and compensate for dimensional tolerances while fixation of the cartridge. The cover for the housing may comprise a viewing window for viewing the cartridge or the volume adapter. Optionally, the wedge or protrusion may engage a cut-out in the volume adapter to axially and rotationally fixate the volume adapter.

### LEGENDS TO THE FIGURES

While the invention has been described in detail in the drawings below and foregoing general description, such description is to be considered illustrative or exemplary and not restrictive. Variations to the disclosed embodiments can be understood and effected by those skilled in the art and practicing the claimed invention, from a study of the drawings, the disclosure, and the appended claims.
Figure 1: A patch injection device comprising a segmented piston rod, a cartridge with a plunger and an interchangeable adapter positioned between the piston rod and the plunger. A pivot bearing between the interchangeable adapter and the piston rod is shown.
Figure 2: Longitudinal section of the injection device according to Figure 1 showing details of the piston rod and the pivot bearing.
Figure 2a: Longitudinal section of the injection device according to Figure 1 showing details for calculating the length of the interchangeable adapter.
Figure 3: Longitudinal section of the injection device according to Figure 1 showing details an alternative interchangeable adapter.
Figure 4: Exploded view of the injection device with an interchangeable adapter.
Figure 5: Exploded view of the injection device with an interchangeable adapter.
Figure 6: Exploded view of the injection device showing the method steps for forming the injection device with an interchangeable adapter.
Figure 7: Exploded view of the injection device showing the method steps for forming the injection device with an interchangeable adapter and a volume adapter.

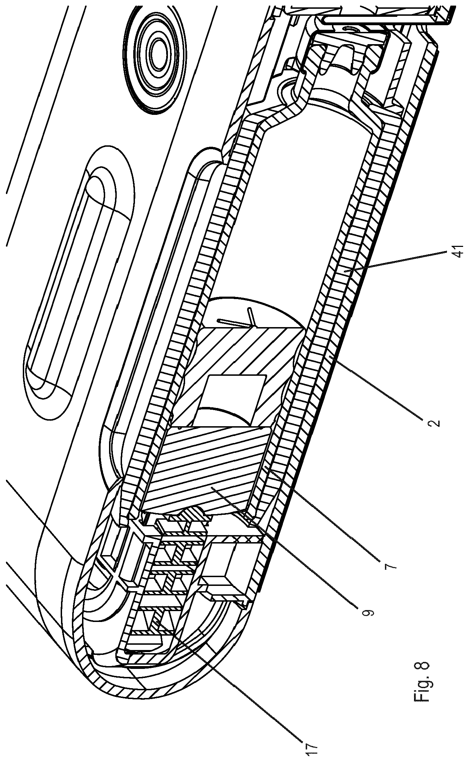

Figure 8: Longitudinal section of an assembled injection device according to Figure 7.

### DETAILED DESCRIPTION OF THE FIGURES

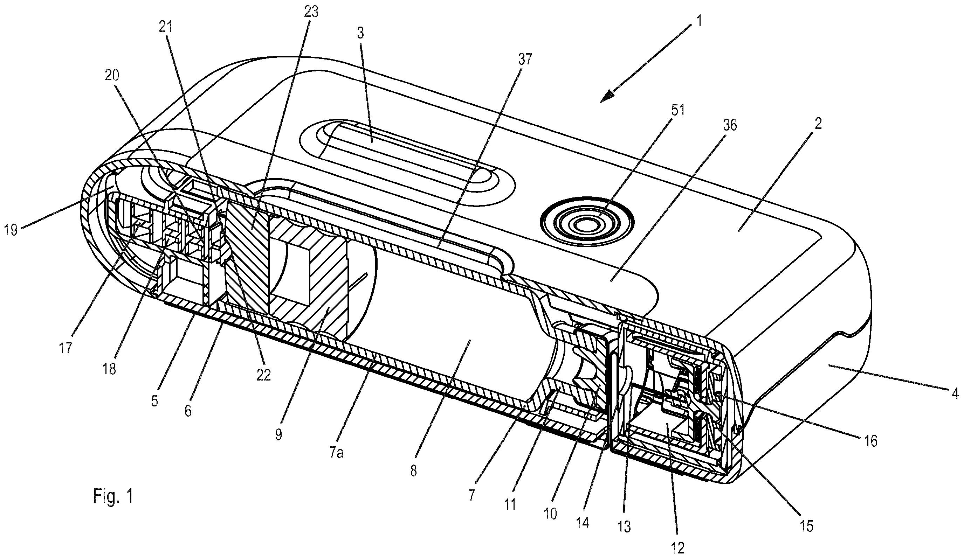

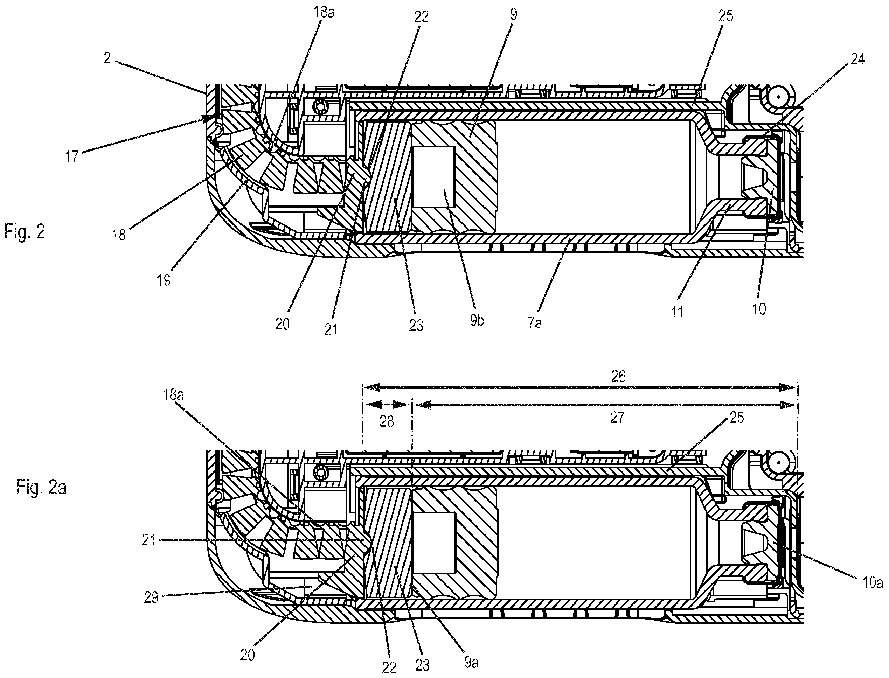

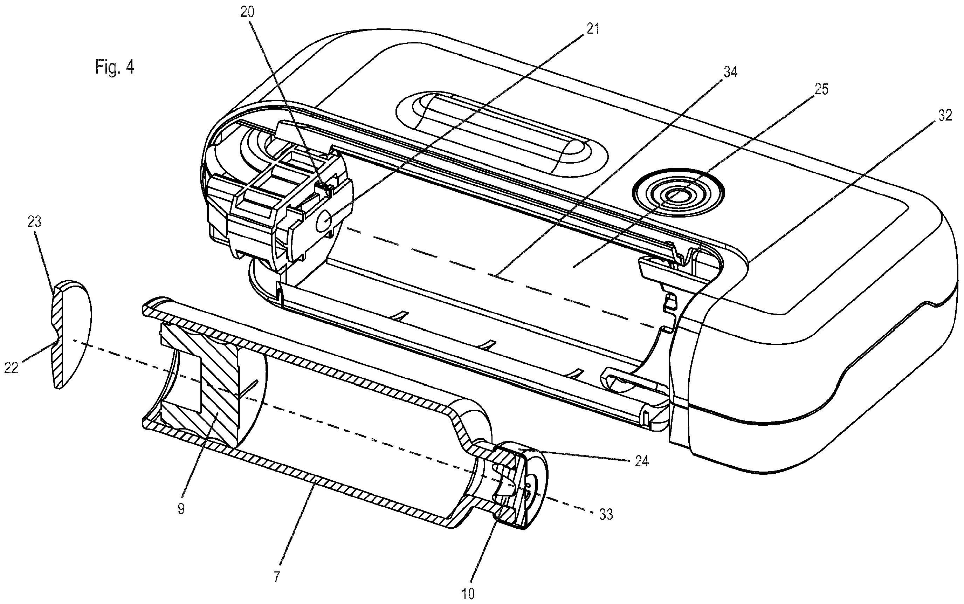

In Figures 1 and 2, an injection device (1) is shown that can be adhered to the skin of the patient. The injection device (1) comprises a housing (2) which has a bottom housing (4) and a skin adhesive layer (5) has been attached to the bottom housing (4). The housing (2) comprises an indicator section (3) that is preferably translucent for an indicator, such as a LED light positioned underneath the indicator section (3) of the housing. A release liner (6) prevents (unwanted) attachment of the injection device (1) to the skin of the patient. The user removes the release liner (6) prior to attaching the device to the skin. The housing (2) is closed by a cover (36) that can only be partly seen in Figure 1, having a viewing window (37) allowing the user to see the contents of a cartridge (7). The cartridge comprises a barrel (7a) which has a cylindrical shape defining a longitudinal axis (33), see Figure 4. The cartridge has a neck portion (11) with an opening that is closed by a septum (10) that is attached to the neck portion (11) using a crimp (24) to form a fluid tight sealing at the distal end of the cartridge (7). A plunger (9), made from an elastic material closes a proximal opening of the barrel (7a) of the cartridge and the medicament (8) is sealed between the septum (10) and the plunger (9). The injection device comprises a fluid path compartment (12) which may be an integral part of the housing (2) or may be a separate housing part housed within the housing (2). The fluid path compartment (12) comprises a spike (13), shaped as a hollow needle and the spike (13) may be driven by a not shown needle insertion mechanism through a passage (14) of the fluid path compartment into the septum (10) of the cartridge. The needle insertion mechanism may use a spring (15) compressed between an inner wall of the housing and the spike (13). The spike (13) and the spring (15) are kept in a retracted position using a retention member (16) and the needle insertion mechanism releases the retention member (16) from the spike, thereby releasing the compressed spring (15) which moves the spike (14) towards and subsequently penetrates the septum. The passage (14) and the septum (10) may be covered by protective films preventing contamination of the septum and fluid path containment during shelflife. The protective films may be removed from the passage (14) and septum (10) prior to use such that the spike does not have to penetrate the protective films before penetrating the septum. The fluid path in the fluid path compartment (12) comprises a tubing connecting the spike (13) to a second needle (that is not shown) which penetrates the skin of the patient when the device has been activated. The fluid path with the spike (13) thereby establishes a fluid connection between the medicament (8) in the cartridge and the patient via the spike (13), the tubing and the skin insertion needle. The skin insertion needle preferably moves perpendicular to the bottom of the housing of the injection device or perpendicular to the longitudinal axis of the cartridge (33).

The injection device (1) may be activated using an activation button (51), after the user has removed the release liner (6) and has attached the device to the skin. The activation button preferably activates the needle insertion mechanism to insert the spike (13) into the cartridge (7) and the needle into the skin. The activation of the needle insertion mechanism is preferably driven by a battery powered electromotor. The needle insertion mechanism may trigger a signal that can be seen by LED lights signaling behind the indicator section (3).

The plunger (9) in the cartridge is advanced using a drive mechanism once the fluid path unit connects the contents of the cartridge to the needle that is configured to penetrate the skin of the patient. The drive mechanism comprises a piston rod (17), that is made from individual segments (18) connected to each other via hinges (18a), which are preferably living hinges. The hinges ensure that the piston rod (17) can bend as the piston rod (17) is guided by a guidance (19) in the housing, see Figure 2. The segmented piston rod (17) starts from a linear, or stacked configuration within the housing and is guided by the housing to make two consecutive 90 degree turns to form a U-shape before returning to the stacked configuration allowing entrance of the barrel of the cartridge.

The last segment (20) forms the distal end of the piston rod (17). The last segment (20) of the piston rod (17) abuts the proximal end (9a) of the plunger (9) in the cartridge (Figure 2a). The last segment (20) has a protrusion (21) pointing in the distal direction and the protrusion (21) forms a bearing with an indention (22) that is present in an interchangeable adapter (23). The interchangeable adapter (23) is positioned between the end of the piston rod (17) and the plunger (9) thus filling the axial space that depends on the position of the last segment (20) of the piston rod and the position of the plunger (9) in the cartridge as the cartridge itself is axially fixated in a cartridge holder (25). Preferably, the position of the last segment (20) is fixed to provide a unified drive mechanism and thus it is the preferred option to change the length of the interchangeable adapter (23) as the position (and thus fill volume) of the plunger (9) in the barrel (7a) is changed.

The protrusion (21) on the last segment of the piston rod (17) preferably has a convex shape that matches a concave shaped recess (22) in the proximal end surface of the interchangeable adapter. The positions of the protrusion and the recess may be changed from the piston rod to the interchangeable adapter. Optionally one of the piston rod (17) or the interchangeable adapter (23) has a flat end surface abutting the protrusion, thus instead of a ball-in-socket bearing, a ball-on-plate bearing is formed.

The calculation for the length of the interchangeable adapter L_{adapter} (28) is explained in Figure 2a. The cartridge holder (25) defines a length L_{cartridge} (26) and from this length (26), the distance between the proximal end (9a) of the plunger (9) and the distal end (10a) of the septum (10) is subtracted, this distance is referenced as d_{plunger} (27) in Figure 2a. Thus L_{adapter} (28) = L_{cartridge} (26) - d_{plunger} (27). Optionally another distance related to a fill/finish safety gap (d_{ff}) is deducted from the L_{cartridge} (26) as well, thus leading to the following formula: L_{adapter} (28) = L_{cartridge} (26) - d_{plunger} (27) - d_{ff}. The fill finish factor is a fixed value related to the parameters for operating the fill/finish line and ensures safe and reliable mounting of the plunger and interchangeable adapter, .ie. the plunger is never flush with the proximal end of the cartridge.

In Figure 2a, a guidance fin (29) for the last segment is presented that abuts and may slide along the inner wall of the barrel (7a) that is part of the cartridge, thereby guiding the last segment into the cartridge.

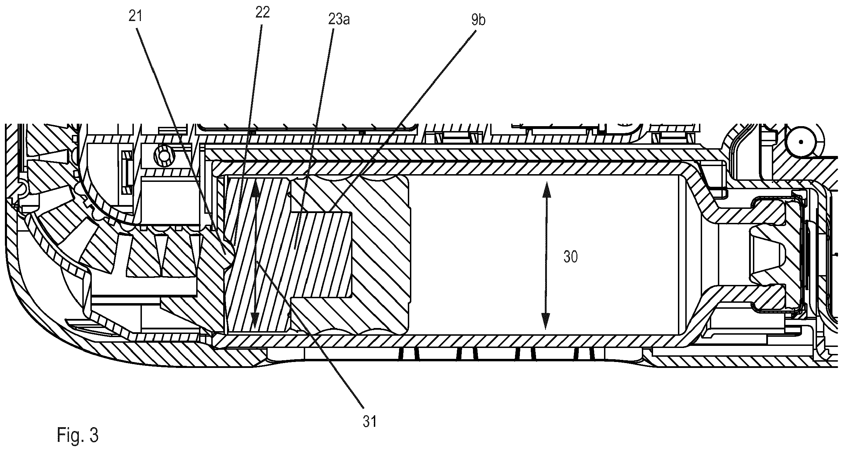

The interchangeable element (23) shown in Figure 2 has a flat distal end surface contacting the proximal end (9a) of the plunger (9). The plunger (9) may have a recess or bore (9b) which may engage a protrusion (23a) of the interchangeable adapter (23) as shown in Figure 3. The engagement between the protrusion of the adapter and the plunger may be a press-fit connection but alternatively a screw type of engagement can be used. The protrusion (23a) enhances the load transfer from the interchangeable adapter (23) to the plunger (9). The external diameter (31) of the interchangeable adapter closely matches the internal diameter (30) of the barrel of the cartridge (Figure 3). A small clearance exists between the adapter and the barrel, preferably, the external diameter (31) of the adapter amounts at least 90% of the internal barrel diameter (30), more preferably 95%.

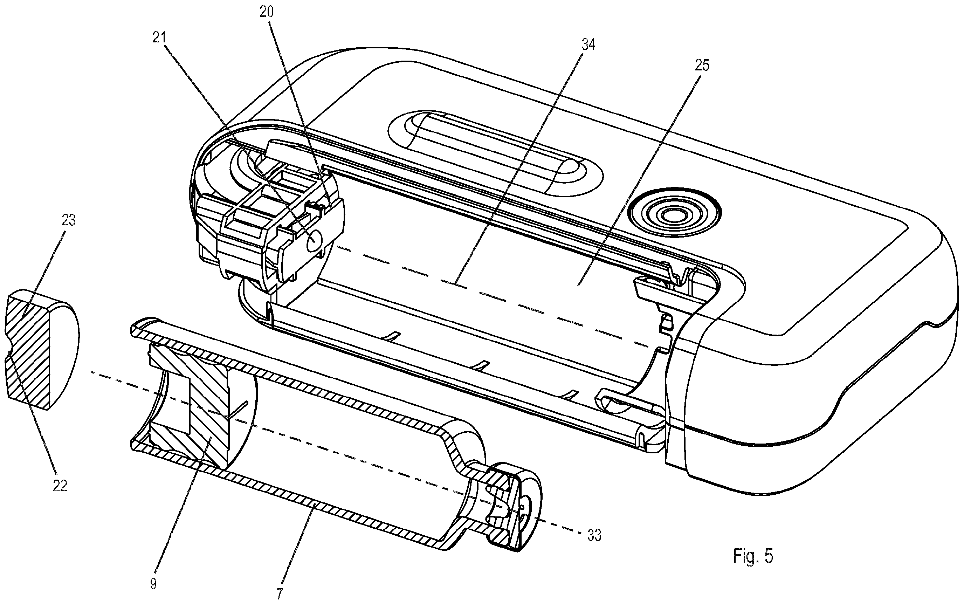

The injection device (1) with a window (32) in the housing (2) is shown in Figure 4. The cartridge holder (25) is present in the window (32) and the cartridge holder defines a longitudinal axis (34) and the center of the axis runs through the protrusion (21) of the last segment (20) of the piston rod (17). The barrel of the cartridge (7) defines a longitudinal axis (33) for the cartridge, which is shown in the exploded view of Figure 4 with an interchangeable adapter (23) having a lower adapter length (L_{adapter}, 28) compared to the arrangement presented in Figure 5. The piston rod (17) is in the retracted position in Figures 4 and 5 such that the cartridge holder (25) is capable for receiving the assembly of the cartridge (7) with the adapter from the side and thereby forming the pivot bearing (21, 22).

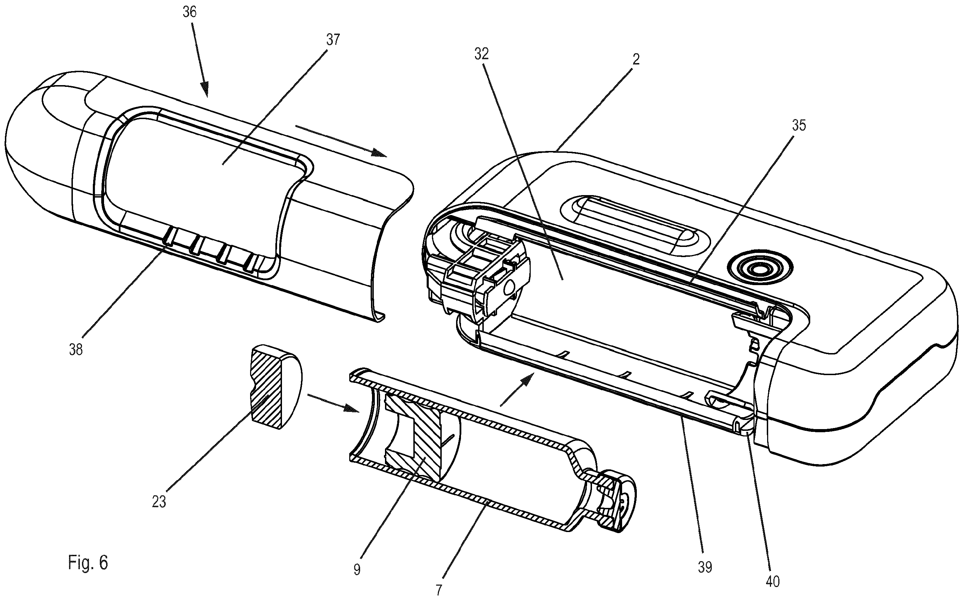

The longitudinal axis for the cartridge (33) and for the cartridge holder (34) are preferably oriented parallel to each other before insertion of the cartridge into the cartridge holder (25) through the window (32) in the housing. The sequence for the cartridge insertion into the injection device is shown in Figure 6. First the interchangeable adapter (23) is selected to match the position of the plunger (9) in the cartridge (7). After insertion of the interchangeable adapter (23), the cartridge is inserted preferably sideways though the window (32) into the cartridge holder (25). The housing (2) is finally closed using a cover (36) which may have a window (37). The housing (2) may have linear guidances (35, 39) that match not shown longitudinal keyways or protrusions present on the inside surface of the cover (36) such that the cover (36) can slide while axially guided parallel to the longitudinal axis (34) of the cartridge holder (25). The cover (36) is axially fixed by a snap-fit connection comprising an aperture (40) in the housing and a not shown flexible arm or protrusion on the inside surface of the cover.

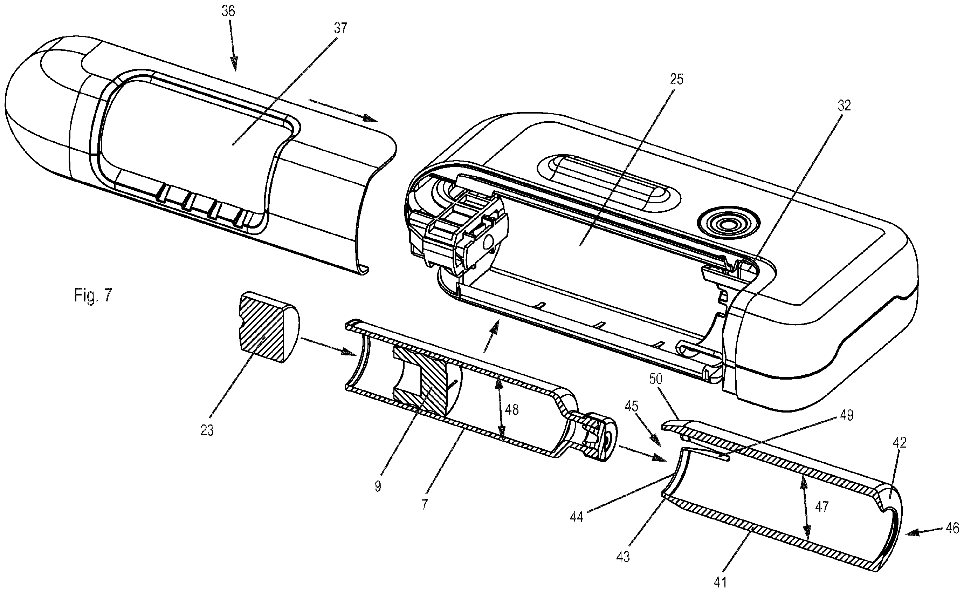

The injection device may additionally benefit from a volume adapter (41) as shown in the exploded view of Figure 7. The volume adapter (41) comprises a hollow cylindrical body having a distal end (42) and a proximal end (43). The distal end (42) has a second opening (46) where a needle can pass through the second end and penetrate the septum of the cartridge received in the volume adapter (41). The proximal end (43) comprises a first opening (45) which can receive the cartridge (7). A flexible arm (44) may be present at the proximal rim of the volume adapter which may irreversibly lock the cartridge (7) in the volume adapter. The volume adapter has an internal hollow space that is available for the cartridge and has an internal diameter (47) that is above the external diameter (48) of the cartridge. Preferably, the interchangeable adapter (23) is first inserted into the proximal end of the cartridge (7) and this subassembly is inserted into the volume adapter (42). The volume adapter (42) is oriented parallel to the cartridge holder and the assembly of the cartridge, the volume adapter and the interchangeable adapter is inserted into the cartridge holder (25) and finally closed by the cover (36).

The inside surface of the cover (36) comprises a protrusion or a wedge that may engage the cartridge (7) or the volume adapter (42) when the cover (36) closes the housing of the injection device. The engagement ensures that the cartridge or the volume adapter holding the cartridge is axially fixated into the cartridge holder. The engagement may also rotationally fixate the cartridge and/or the volume adapter. The engagement preferably comprises plastic deformation of the wedge or protrusion on the cover by the proximal rim of the cartridge (7) or the proximal rim of the volume adapter. The volume adapter may have a V-shaped cut-out (49) and/or a facet (50) at the proximal end which engage a protrusion or wedge present on the inside surface of the cover. The facet (50) or V-shaped cut-out (49) may have a sloped counter surface matching a sloped surface on the inside of the cover thereby gradually densifying and deforming the material from the cover (30) (or vice versa the material from the volume adapter) as the cover slides towards its end position on the housing. Preferably the cover (36) is made from a plastic material with a lower hardness or yield strength compared to the material of the cartridge or the volume adapter. The cover may have markings (38) on the outside surface allowing to follow the delivery of the medicament during use.

**PART ANNOTATION**

| | | | |
|---|---|---|---|
| 1 | Injection device | 25 | Cartridge holder |
| 2 | Housing | 26 | Length cartridge holder L_{cartridge} |
| 3 | Indicator section | 27 | Distance plunger to septum, |
| 4 | Bottom housing | | d_{plunger} |
| 5 | Skin adhesive layer | 28 | Length adapter, L_{adapter} |
| 6 | Release liner | 29 | Guidance fin last segment |
| 7 | Cartridge | 30 | Internal diameter barrel |
| 7a | Barrel | 31 | External diameter |
| 8 | Medicament | | interchangeable element |
| 9 | Plunger | 32 | Window in housing |
| 9a | Proximal end plunger | 33 | Longitudinal axis cartridge |
| 9b | Recess plunger | 34 | Longitudinal axis cartridge |
| 10 | Septum | | holder |
| 10a | Distal end septum | 35 | Guidance |
| 11 | Neck | 36 | Cover |
| 12 | Fluid path compartment | 37 | Window in cover |
| 13 | Spike, needle | 38 | Marking |
| 14 | Passage | 39 | Guidance |
| 15 | Spring | 40 | Snap fit connector, aperture |
| 16 | Retention member | 41 | Volume adapter |
| 17 | Piston rod | 42 | Distal end |
| 18 | Segment | 43 | Proximal end |
| 18a | Hinge | 44 | Flexible retaining arm |
| 19 | Guidance housing | 45 | First open end |
| 20 | Last segment, distal end piston rod | 46 | Second open end |
| | | 47 | Inner diameter volume adapter |
| 21 | Protrusion last segment | 48 | Outer diameter cartridge |
| 22 | Indention, recess adapter | 49 | V-shaped cut-out |
| 23 | Interchangeable adapter | 50 | Facet |
| 23a | Protrusion interchangeable adapter | 51 | Activation button |
| 24 | Crimp | | |

## Claims

1. An injection device (1) comprising:
a housing (2) comprising a cartridge holder (25),
a cartridge (7) received in the cartridge holder (25) and containing a medicament (8) sealed in a barrel (7a) defining a longitudinal axis (33) and the ends of the barrel are closed by a moveable plunger (9) and a pierceable septum (10),
a drive assembly comprising a curved piston rod (17) configured for advancement of the plunger (9) in the cartridge along the longitudinal axis (33) from a retracted position to an extended position for expelling the medicament (8) from the injection device (1),
an interchangeable adapter (23) positioned between the curved piston rod (17) and the plunger (9) for transferring the load from the piston rod to the plunger during advancement,
wherein a pivot bearing (21,22) is formed between the end (20) of the piston rod (17) and the interchangeable adapter (23).

2. The injection device according to claim 1, further comprising a volume adapter (41) located in the cartridge holder (25) of the injection device (1) wherein the cartridge holder (25) defines a first space having a first volume, the volume adapter (41) comprising:
- a body, preferably cylindrically shaped defining a second space to be arranged within the first volume of the cartridge holder (25),
- a hollow interior, preferably cylindrically shaped defining a third space having a third volume that is below the first volume wherein the cartridge (7) is received within the hollow interior.

3. The injection device according to claim 2, wherein the volume adapter (41) comprises two open ends, a first open end (45) for receiving the cartridge (7) and a second open end (46), located opposite to the first open end, defining a passage for a needle.

4. The injection device according to any of the previous claims wherein the piston rod (17) is a segmented piston rod having connectors (18a) between the individual segments (18) allowing for relative rotational movement between the individual segments (18), preferably the connector (18a) is an integral hinge or living hinge.

5. The injection device according to any of the previous claims wherein the interchangeable adapter (23) has a length (28) calculated from the length of the cartridge holder (26) deducted by the distance (27) between the proximal end of the plunger (9a) and the distal end (10a) of the pierceable septum (10).

6. The injection device according to any of the previous claims wherein the interchangeable adapter (23) has a cylindrical shape with an outer diameter (31) that is below the inner diameter of the barrel (30), preferably having an outer diameter that is at least 90% of the inner diameter of the barrel, more preferably at least 95% of the inner diameter.

7. The injection device according to any of the previous claims wherein the interchangeable adapter (23) has a front end configured for engaging with or connecting to the plunger (9) in the cartridge.

8. The injection device according to any of the previous claims wherein the pivot bearing (21, 22) is formed between the end (20) of the piston rod and a back end of the interchangeable adapter (23).

9. The injection device according to any of the previous claims wherein the pivot bearing (21, 22) comprises a protrusion (21) present at the end of the piston rod (20) or on the back end of the interchangeable adapter, the protrusion preferably having a convex shape.

10. The injection device according to any of the previous claims wherein the pivot bearing (21, 22) comprises at least one flat surface present on the piston rod (17) or the interchangeable adapter (23).

11. The injection device according to any of the previous claims wherein the pivot bearing (21, 22) comprises one convex shaped protrusion present on one of the piston rod (17) or the interchangeable adapter (23) that is matched with a complementary concave shaped recession present on the other one of the piston rod (17) or the interchangeable adapter (23).

12. A method for assembling of a range of an injection devices or an infusion devices having cartridges with different preset medicament fill volumes comprising the steps of:
- providing a housing (2) comprising a cartridge holder (25) located behind a window (32) in the housing, the housing (2) further enclosing a drive assembly for advancing an at least partially curved piston rod (17) from a retracted position to an extended position, wherein the retracted position is defined by the axial position of the end of the piston rod (20) with respect to the housing (2),
- providing a cartridge (7) containing a preset volume of a medicament sealed in a barrel (7a) defining a first longitudinal axis (34) and the ends of the barrel are closed by a pierceable septum (10) and a plunger (9) that can be moved along the first longitudinal axis (34) towards the pierceable septum (10) by advancing the piston rod,
- providing an interchangeable adapter (23) having a defined length (28) and which is selected from a range of interchangeable adapters having different lengths, the interchangeable adapter (23) is selected according to the preset medicament volume of the cartridge,
- inserting the interchangeable adapter (23) into the cartridge (7) whereby a front end of the interchangeable adapter (23) engages the plunger (9) in the cartridge (7),
- inserting the cartridge (7) comprising the interchangeable adapter (23) through the window (32) into the cartridge holder (25) to form a pivot bearing (21, 22) between the back end of the adapter and the end (20) of the piston rod (17),
- closing the window (32) in the housing (2) with a cover (36).

13. The assembly method according to the previous claim wherein prior to inserting the cartridge (7) comprising the interchangeable adapter (23) through the window (32) into the cartridge holder (25) a further method step is introduced:
- inserting the cartridge (7) containing the preset volume of medicament (8) in a volume adapter (41) which is configured to be inserted in the cartridge holder (25) which defines a first space having a first volume, the volume adapter (41) comprising:
- a monolithic body, preferably cylindrically shaped defining a second space that fits within the first space having the first volume of the cartridge holder,
- a hollow interior, preferably cylindrically shaped defining a third space having a third volume that is below the first volume wherein the cartridge (7) containing the preset volume is received within the hollow interior or coaxially guided by the volume adapter.

14. The assembly method according claims 12 or 13 wherein the cartridge holder (25) has a longitudinal shape defining a second longitudinal axis (34) and wherein the cartridge comprising the interchangeable adapter or the volume adapter is oriented with its first longitudinal axis (33) parallel to the second longitudinal axis (34) and is inserted through the window (32) perpendicular to the first and second longitudinal axis.

15. The assembly method according to claim 14 wherein the cover (36) is moved parallel to the second longitudinal axis (34) upon closing the window (32) in the housing and wherein the housing (2) and the cover (36) have engagement means for guiding the cover (36) during closing of the window in the housing,

16. The assembly method according to claims 12 to 15 wherein the cover (36) comprises a protrusion or wedge that is deformed by the barrel of the cartridge (7) or volume adapter (41) when the window (32) is closed by the cover (36) thereby fixating the cartridge (7) or volume adapter (41) in the cartridge holder (25) along the second longitudinal axis (34).
